# DEMANDE DE BREVET EUROPEEN

(11) **EP 0 785 219 A1**
(43) Date de publication de la demande: **23.07.1997**
(21) Numéro de dépôt: 97400055.6
(22) Date de dépôt: 13.01.1997
(51) Int. Cl.: C08F 8/32, C07C 51/567, C10L 1/10, C10M 133/00, C10M 149/06

(54) **Procédé simplifié de fabrication d'alkenyl succinimides ou de polyalkenyl succinimides**

(30) Priorité: 18.01.1996 FR 9600645
(71) Demandeur: Institut Français du Pétrole, 92852 Rueil-Malmaison Cedex (FR)
(72) Inventeur: Binet, Daniel, 92500 Rueil Malmaison (FR); Paille, Fabrice, 94100 Saint Maur des Fosses (FR); Gateau, Patrick, 78310 Maurepas (FR); Durand, Jean-Pierre, 78400 Chatou (FR)

(57) **Abrégé**

On décrit un procédé de préparation d'un alkényl ou d'un polyalkénylsuccinimide comprenant successivement; une étape (a) dans laquelle on prépare un anhydride alkényl ou polyalkénylsuccinique par réaction de ène-synthèse entre au moins une oléfine ou une polyoléfine et au moins un anhydride d'acide dicarboxylique insaturé choisi parmi l'anhydride maléïque et les anhydrides maleïques substitués par 1 ou 2 groupes méthyles, ladite réaction étant réalisée au sein d'au moins un solvant aromatique choisi parmi le toluène et les xylènes; une étape (b) dans laquelle on fait réagir le produit de l'étape (a) avec au moins un alcool ou une amine; et une étape (c) dans laquelle on fait réagir le produit obtenu dans l'étape (b) avec une amine.

Ces succinimides peuvent être utilisés comme additifs détergents pour les carburants moteurs et comme additifs dispersants sans cendres pour les lubrifiants moteurs.

## Description

La présente invention concerne un procédé simplifié de fabrication d'alkényl-ou de polyalkénylsuccinimides exempts de chlore.

Dans le domaine des additifs pour produits pétroliers, notamment des additifs détergents pour carburants moteurs et des dispersants pour lubrifiants moteurs, il est souvent fait appel, pour synthétiser les composés amphiphiles recherchés dans ces applications, à des polyisobutènes fonctionnalisés, le plus souvent à l'aide d'anhydride maléïque. Les anhydrides polyisobuténylsucciniques considérés sont préparés suivant deux procédés principaux.

Le premier consiste en la condensation d'anhydride maléïque sur du polyisobutène suivant une réaction de ène-synthèse. Cette réaction nécessite une température élevée et des temps de réaction tels que, dans ces conditions, apparaissent des résines résultant de la polymérisation de l'anhydride maléïque. Ces produits secondaires obligent à une étape de filtration, rendue très délicate par la consistance de ces produits. Des améliorations visant à diminuer la quantité de résines ont été proposées. Elles consistent le plus souvent à effectuer la réaction en présence d'inhibiteurs de polymérisation radicalaire, comme dans les documents DE-A-1 102 142, DE-A-3 320 468 et US-A-3 476 774; de composés halogénés comme dans les documents GB-A-1 356 802, GB-A-1 480 453 (= FR-A-2 273 014), US-A-3 960 900, DE-A-3 320 468 déjà cité, US-A-4 278 604, et US-A-4 255 340; de sels métalliques, comme dans les documents GB-A-2 081 274 et WO-A-082/00467; ou de peroxydes comme dans le document US-A-4 599 432 (=FR-A-2 555 595). Une autre méthode, décrite par exemple dans le document US-A-4 496 746, consiste à faire réagir l'anhydride maléïque en émulsion dans un solvant inerte, ceci grâce à l'utilisation d'un tensioactif. Dans tous les cas, les produits secondaires ne sont jamais totalement éliminés.

Dans un second type de procédé, on réalise d'abord une chloration du polyisobutène suivie de la condensation du polyisobutène chloré et d'anhydride maléïque. Ce procédé concurrence le précédent grâce à un abaissement de la température de réaction, à une meilleure conversion et à l'absence de réactions secondaires conduisant à des résines insolubles. Toutefois, ce procédé conduit habituellement à des produits contenant une certaine quantité de chlore résiduel, ce qui les exclut d'un nombre d'applications de plus en plus important, compte tenu des spécifications de plus en plus sévères concernant les teneurs en chlore des additifs pour produits pétroliers.

Ces anhydrides polyisobuténylsucciniques sont ensuite modifiés le plus souvent par des amines et doivent donc être débarrassés de toute trace d'anhydride maléïque libre. Pour ce faire, une étape d'évaporation sous vide est nécessaire.

La présente invention propose un procédé simplifié de fabrication d'alkényl- ou de polyalkénylsuccinimides exempts de chlore, qui ne met pas en jeu de réaction en émulsion ou en suspension et dans lequel les produits secondaires sont totalement éliminés, ce qui évite une étape de filtration longue, délicate et coûteuse. De plus, le procédé décrit dans la présente invention ne nécessite pas l'élimination de l'anhydride maléïque n'ayant pas réagi pour la préparation d'additifs pour produits pétroliers exempts de chlore, notamment d'additifs détergents pour carburants moteurs.

Le procédé décrit dans la présente invention comprend une étape de fabrication d'anhydrides alkényl- ou polyalkénylsucciniques par réaction entre une oléfine ou une polyoléfine et un anhydride d'acide dicarboxylique insaturé, dans des conditions de ène-synthèse et en solution dans au moins un solvant aromatique choisi parmi le toluène et les xylènes, utilisé en une proportion entre 30 et 70 % en masse du mélange réactionnel.

Les oléfines et polyoléfines mises en jeu peuvent avoir une masse moléculaire moyenne en nombre comprise entre 400 et 10 000 et de préférence entre 400 et 3000. Les polyoléfines considérées consistent plus particulièrement en des polyisobutènes présentant une teneur en doubles liaisons "exo" supérieure à 50 %. Ils sont obtenus de préférence par polymérisation de l'isobutène en présence d'un catalyseur ne contenant pas de chlore, tel que par exemple le trifluorure de bore. L'anhydride d'acide carboxylique insaturé est choisi plus particulièrement dans le groupe formé par l'anhydride maléïque et les anhydrides maléïques substitués par un ou deux groupes méthyles sur les atomes de carbone de la liaison éthylénique, à savoir l'anhydride citraconique (méthylmaléïque) et l'anhydride pyrocinchonique (diméthylmaléïque). De préférence, on utilise l'anhydride maléïque.

Une des caractéristiques essentielles du procédé de l'invention est la mise en oeuvre de la réaction de ène-synthèse au sein d'un solvant aromatique choisi parmi le toluène et les xylènes, à une teneur comprise entre 30 et 70% en masse, de préférence de 35 à 60 % en masse et par exemple de 50 % en masse par rapport au mélange réactionnel.

Par ailleurs, la réaction est conduite à une température comprise entre 150 et 300°C, de préférence entre 180 et 250°C, par exemple sous pression autogène. En général, la pression développée lors de la réaction est comprise entre 2 et 40 bars, plus particulièrement entre 3 et 10 bars.

L'anhydride maléïque ou maléïque substitué est en général utilisé dans un rapport molaire de 0,5 à 2, de préférence de 1 à 1,1, par rapport à l'oléfine ou à la polyoléfine (le plus souvent le polyisobutène).

Les anhydrides alkényl- et polyalkénylsucciniques (en particulier polyisobuténylsucciniques) sont obtenus par le procédé de l'invention, avec des rendements élevés, qui peuvent atteindre 85 % et ne contiennent pas de produits secondaires.

Le procédé de la présente invention comprend une seconde étape ayant pour objectif d'éviter toute purification du produit obtenu lors de la première étape. L'anhydride maléïque n'ayant pas réagi lors de la ène-synthèse est modifié par des composés tels que des alcools ou des amines, afin de le solubiliser et de diminuer sa réactivité vis à vis des amines utilisées lors de la synthèse des additifs détergents pour carburants moteurs ou d'additifs dispersants sans cendres pour lubrifiants moteurs.

Les alcools utilisés sont plus particulièrement des polypropylèneglycols, des copolymères d'oxyde d'éthylène et d'oxyde de propylène ou des monoéthers de polypropylèneglycols.

Les amines utilisées sont des polyoxyalkylène-amines, des alkénylamines ou encore des polyalkénylsuccinimides à fonction amine primaire.

Les alcools ou les amines sont introduits dans le mélange réactionnel issu de la première étape du procédé dans un rapport molaire compris entre 1 et 1,5 par rapport à l'anhydride maléïque résiduel présent. La réaction est conduite à une température comprise entre l'ambiante et la température d'ébullition du solvant.

Dans une troisième étape, le mélange réactionnel issu de la seconde étape du procédé est modifié de manière connue, par réaction avec des amines pour former des imides.

Les amines utilisées dans cette étape sont plus particulièrement des polyéthylène-polyamines telles que la triéthylène-tétramine, ou la tétraéthylène-pentamine, utilisées par exemple en une proportion molaire de 0,5 à 1 par rapport à l'anhydride alkényl- ou polyalkénylsuccinique (en particulier polyisobutényl-succinique). La réaction est effectuée en général à la température d'ébullition du solvant aromatique utilisé.

Les produits tels qu'obtenus par le procédé décrit ci-dessus constituent également un objet de l'invention. Ils peuvent être utilisés comme additifs détergents pour les carburants et, dans ce cas, ils peuvent être utilisés conjointement à une huile porteuse, telle qu'un polypropylèneglycol ou un éther de polypropylèneglycol. Ils peuvent également être utilisés comme additifs dispersants sans cendres pour les lubrifiants moteurs.

Les exemples suivants illustrent l'invention.

### Exemple 1

Dans un autoclave équipé d'un système d'agitation, on introduit :
250 g de polyisobutène de masse moléculaire moyenne en nombre voisine de 1000 et dont la composition des doubles liaisons terminales, déterminée par RMN du proton est la suivante :
- 90 % de doubles liaisons "exo" ;
- 10 % de doubles liaisons "endo" ;
   24,5 g d'anhydride maléïque ;
   et 147,8 g de xylènes, soit 35 % en masse du mélange réactionnel.

Le mélange est dégazé par balayage d'azote à température ambiante. Il est ensuite agité pendant 15 heures à 200°C. La pression développée lors de la réaction est de 2,2 bars. Aucun produit secondaire n'est formé. Sur une fraction du mélange réactionnel, le solvant et l'anhydride maléïque n'ayant pas réagi sont éliminés par distillation sous vide. L'indice d'anhydride du produit obtenu (nombre de moles d'anhydride pour 100 g de produit) est de 0,067. La concentration en anhydride maléïque libre dans le mélange réactionnel est déterminée par chromatographie d'exclusion stérique. Cette concentration est de 1,64 % masse.

A 400g du mélange réactionnel ci-dessus, on ajoute sous agitation à la température ambiante, 71,7 g d'un polypropylèneglycol de masse moléculaire voisine de 1000 et 38,6 g de xylènes. La solution est portée au reflux du solvant pendant 3 heures.

Après refroidissement, on ajoute sous agitation 12,4 g de xylènes et 23 g de tétraéthylène-pentamine. Le mélange réactionnel est porté au reflux du solvant pendant 5 heures. Environ 3 cm³ d'eau sont éliminés. La solution obtenue a une teneur en azote de 1,6 % en masse.

### Exemple 2

La première étape de l'exemple 1 est répétée en portant la température de réaction à 220°C. La pression développée pendant la réaction est alors de 5 bars. Le produit obtenu a un indice d'anhydride de 0,075. La solution réactionnelle contient 1,1 % en masse d'anhydride maléïque n'ayant pas réagi.

Les deuxièmes et troisièmes étapes du procédé sont conduites dans des conditions identiques à celles de l'exemple 1, en adaptant les quantités de polypro-pylèneglycol et de tétraéthylène-pentamine à l'indice d'anhydride du produit et à l'anhydride libre présent. Ainsi, pour 400 g de mélange réactionnel, on ajoute 48,3 g de polypropylèneglycol puis 25,9 g de tétraéthylène-pentamine. La solution obtenue a une teneur en azote de 1,9 % en masse.

### Exemple 3

Si, dans l'exemple 2, toutes choses étant égales par ailleurs, on remplace le polypropylèneglycol par 99,4 g d'une solution contenant 70% masse d'une poly-isobutényl-monosuccinimide obtenue par réaction entre un anhydride polyisobutényl-succinique d'indice d'anhydride égal à 0,075 et d'une triéthylène-tétramine dans un rapport molaire anhydride / polyéthylène-amine de 1, la solution obtenue a une teneur en azote de 2.2 % en masse.

### Exemple 4

Si, dans l'exemple 1, toutes choses étant égales par ailleurs, on remplace le polypropylène glycol par 14 g de polypropylèneglycol-diamine de masse moléculaire moyenne voisine de 400, la solution obtenue a une teneur en azote de 2,07 % masse.

### Exemple 5 (comparatif)

Si, dans l'exemple 1, toutes choses étant égales par ailleurs, la réaction est conduite en présence de 91,5 g de xylènes, soit 25 % en masse des réactifs utilisés, on constate la formation de produits secondaires huileux et bruns. La présence de ces produits insolubles interdit toute utilisation du produit sans purification préalable.

### Exemple 6 (comparatif)

Dans un autoclave équipé d'un système d'agitation, on introduit :
250 g du polyisobutène de l'exemple 1 ;
24,5 g d'anhydride maléïque ;
et 147.8 g de xylènes, soit 35 % en masse du mélange réactionnel.

Le mélange est dégazé par balayage d'azote à température ambiante. Il est ensuite agité pendant 15 heures à 200°C. Le produit obtenu a un indice d'anhydride de 0,067.

A 400 g du mélange réactionnel obtenu, on ajoute à température ambiante et sous agitation 12,4 % de xylènes et 23 g de tétraéthylène-pentamine. Le mélange est agité pendant 5 heures au reflux du solvant. Après refroidissement, on constate la présence de produit insoluble dans le milieu réactionnel, ce qui interdit toute utilisation du produit. La quantité de produit insoluble présent dans le milieu, évaluée par dilution avec du xylène, filtration et séchage, est de 0,65 % en masse.

### Exemple 7 : Test sur moteur Diesel

Un test d'encrassement des injecteurs sur moteur XUD9 est effectué. Le carburant utilisé est un gazole dont les caractéristiques sont détaillées dans le Tableau 1. La tendance à l'encrassement est donnée par le débit résiduel moyen des quatre injecteurs, obtenu après 6 heures de fonctionnement du moteur. Les essais sont réalisées comparativement au gazole seul, en additionnant celui-ci de 500 ppm des solutions obtenues dans les exemples 1 et 2.

**Tableau 1**

| | |
|---|---|
| Masse volumique | 837,2 kg/m³ |
| Teneur en soufre | 0,045 %masse |
| Distillation | |
| Point initial | 204,5°C |
| 5 % | 240,0°C |
| 10 % | 253,0°C |
| 20 % | 269,5°C |
| 30 % | 280,5°C |
| 40 % | 287,5°C |
| 50 % | 293,0°C |
| 60 % | 298,0°C |
| 70 % | 303,5°C |
| 80 % | 309,5°C |
| 90 % | 321,0°C |
| 95 % | 336,5°C |
| point final | 349,0°C |

Les résultats sont rassemblés dans le Tableau 2 et montrent l'efficacité des produits obtenus selon l'invention.

**Tableau 2**

| | | | |
|---|---|---|---|
| **Résultats d'encrassement d'injecteurs (XUD9 - 6 heures)** | | | |
| Levée d'aiguilles (mm) Débit résiduel moyen (%) | 0,1 | 0,2 | 0,3 |
| Gazole (référence) | 3,7 | 28,4 | 50,0 |
| Gazole + 500 ppm d'additif de l'exemple 1 | 45,2 | 60,3 | 69,1 |
| Gazole + 500 ppm d'additif de l'exemple 2 | 49,0 | 65,2 | 73,6 |
| Gazole + 500 ppm d'additif de l'exemple 3 | 59,7 | 74,3 | 79,9 |

### Exemple 8 : Test sur moteur à essence

Des formulations à base de l'additif de l'exemple 1 et de polypropylèneglycol de masse moléculaire voisine de 1000 déjà utilisé lors des synthèses sont préparées. Des essais moteurs sont réalisés afin d'évaluer l'influence des additifs sur les quantités de dépôts au niveau des soupapes d'admission.

La méthode d'essai utilise un moteur Mercedes M 102 E et elle fonctionne de façon cyclique selon un schéma porte-à-porte durant 60 heures. Le carburant de référence utilisé est un carburant sans plomb ayant un indice d'octane "Recherche" de 96,8 et dont les caractéristiques sont détaillées dans le Tableau 3.

Les essais sont menés en présence de différentes concentrations d'additif et de polypropylèneglycol et comparés à un essai en l'absence d'additif. Les résultats obtenus (Tableau 4) sont exprimés en poids (grammes) de dépôts sur les tulipes des soupapes d'admission. Ils montrent l'efficacité des additifs obtenus suivant l'invention.

**Tableau 3**

| | |
|---|---|
| Masse volumique à 15°C | 754,0 kg/m³ |
| Pression de vapeur Reid | 618 hPa |
| Distillation | |
| Point initial | 30,7°C |
| 5 % | 45,0°C |
| 10 % | 51,5°C |
| 20 % | 65,5°C |
| 30 % | 80,5°C |
| 40 % | 97,0°C |
| 50 % | 111,0°C |
| 60 % | 123,0°C |
| 70 % | 138,0°C |
| 80 % | 154,5°C |
| 90 % | 172,8°C |
| 95 % | 185,0°C |
| Point final | 205,0°C |

**Tableau 4**

| **Essai M 102 E - Quantité de dépôts (en grammes)** | | | | | | | |
|---|---|---|---|---|---|---|---|
| Additif de l'exemple 1 (ppm) | PPG (ppm) | Soupape n°1 | Soupape n°2 | Soupape n°3 | Soupape n°4 | Total | Dépôt moyen/soupape |
| 0 | 0 | 0,278 | 0,479 | 0,186 | 0,188 | 1,131 | 0,283 |
| 130 | 200 | 0,005 | 0,011 | 0,043 | 0,001 | 0,060 | 0,015 |
| 400 | 0 | 0,036 | 0,002 | 0,038 | 0,034 | 0,110 | 0,028 |
| 400 | 174 | 0,014 | 0,005 | 0,001 | 0,015 | 0,035 | 0,009 |

## Revendications

1. Procédé simplifié de préparation d'un alkényl- ou d'un polyalkénylsuccinimide caractérisé en ce qu'il comprend successivement une étape (a) dans laquelle on prépare un anhydride alkényl- ou polyalkénylsuccinique par réaction de ène-synthèse entre au moins une oléfine ou une polyoléfine et au moins un anhydride d'acide dicarboxylique insaturé choisi parmi l'anhydride maléïque et les anhydrides maleïques substitués par 1 ou 2 groupes méthyles, caractérisé en ce que ladite réaction est réalisée au sein d'au moins un solvant aromatique choisi parmi le toluène et les xylènes en une proportion comprise entre 30 et 70 % en masse par rapport au mélange réactionnel, dans laquelle une fraction dudit anhydride d'acide dicarboxylique insaturé ne réagit pas avec ladite oléfine ou polyoléfine ; une étape (b) dans laquelle on fait réagir le produit de l'étape (a), contenant l'anhydride d'acide dicarboxylique insaturé qui n'a pas réagi, avec au moins un alcool ou une amine; et une étape (c) dans laquelle on fait réagir le produit obtenu dans l'étape (b) avec une amine.

2. Procédé selon la revendication 1, caractérisé en ce que ladite oléfine ou polyoléfine a une masse moléculaire moyenne en nombre comprise entre 400 et 10 000.

3. Procédé selon l'une des revendications 1 et 2, caractérisé en ce que ladite polyoléfine est un polyisobutène présentant une teneur en doubles liaisons "exo" supérieure à 50 %.

4. Procédé selon l'une des revendications 1 à 3, caractérisé en ce que la proportion de solvant aromatique mis en jeu dans l'étape (a) est de 35 à 60 % en masse par rapport au mélange réactionnel.

5. Procédé selon l'une des revendications 1 à 4, caractérisé en ce que la réaction de l'étape (a) est conduite à une température de 150 à 300°C.

6. Procédé selon l'une des revendications 1 à 5, caractérisé en ce que l'anhydride maléïque ou maléïque substitué est mis en jeu dans l'étape (a) dans un rapport molaire de 0,5 à 2 avec l'oléfine ou la polyoléfine.

7. Procédé selon l'une des revendications 1 à 6, caractérisé en ce que, dans l'étape (b), l'alcool mis en jeu est au moins un propylèneglycol, un copolymère d'oxyde d'éthylène et d'oxyde de propylène ou un monoéther de polypropylèneglycol et/ou l'amine mise en jeu est au moins une polyoxyalkylène-amine, une alkénylamine ou un polyalkénylsuccinimide à fonction amine primaire.

8. Procédé selon l'une des revendications 1 à 7, caractérisé en ce que, dans l'étape (b), l'alcool ou l'amine est mis en jeu dans un rapport molaire de 1 à 1,5 par rapport à l'anhydride maléïque ou maléïque substitué résiduel présent.

9. Procédé selon l'une des revendications 1 à 8, caractérisé en ce que, dans l'étape (b), la température de réaction va de la température ambiante jusqu'à la température d'ébullition du solvant.

10. Procédé selon l'une des revendications 1 à 9, caractérisé en ce que, dans l'étape (c), l'amine est une polyéthylène-polyamine choisie parmi la triéthylène-tétramine et la tétraéthylène-pentamine.

11. Procédé selon l'une des revendications 1 à 10, caractérisé en ce que la polyéthylène-polyamine est mise en jeu en une proportion molaire de 0,5 à 1 par rapport à l'anhydride alkényl- ou polyalkénylsuccinique formé dans l'étape (a).

12. Procédé selon l'une des revendications 1 à 11, caractérisé en ce que, dans l'étape (c), la réaction est effectué à température d'ébullition du solvant.

13. Composition obtenue par un procédé selon l'une des revendications 1 à 12.

14. Utilisation d'une composition selon la revendication 13 comme additif détergent pour carburants.

15. Formulation d'additifs pour carburants comprenant une composition selon la revendication 13 et un polypropylèneglycol ou un éther de polypropylèneglycol.

16. Utilisation d'une composition selon la revendication 13 comme additif dispersant sans cendres pour lubrifiants.
